# EUROPEAN PATENT APPLICATION

(11) **EP 4 468 311 A1**
(43) Date of publication of application: **27.11.2024**
(21) Application number: 23740362.1
(22) Date of filing: 16.01.2023
(51) Int. Cl.: G16H 50/20

(54) **STATISTICAL DATA ACQUISITION DEVICE, DEGREE OF CONTRIBUTION CALCULATION DEVICE, TREATMENT ACTION SEARCH DEVICE, TREATMENT OBJECT SEARCH DEVICE, STATISTICAL DATA ACQUISITION PROGRAM, DEGREE OF CONTRIBUTION CALCULATION PROGRAM, TREATMENT ACTION SEARCH PROGRAM, AND TREATMENT OBJECT SEARCH PROGRAM**

(30) Priority: 17.01.2022 JP 2022005265
(71) Applicant: Aizoth Inc., Tsukuba-shi, Ibaraki 305-0031 (JP)
(72) Inventor: KAWAJIRI Kotaro, Tsukuba-shi, Ibaraki 305-0031 (JP)
(74) Representative: Gille Hrabal Partnerschaftsgesellschaft mbB Patentanwälte
(86) International application number: PCT/JP2023/001058
(87) International publication number: WO 2023/136354

(57) **Abstract**

A learner (26) is trained so as to predict and output a result of a treatment action on a treatment object from attribute information representing an attribute of the treatment object and treatment information representing the content of the treatment action on the treatment object, on the basis of a training data group (16). A statistical data acquisition unit (32) inputs to the trained learner (26) an input data group (38) that includes a plurality of input data (40) including input attribute information (42) representing an attribute of the treatment object and input treatment information (44) representing the content of a treatment action on the treatment object, the input attribute information (42) being mutually different and the input treatment information (44) being mutually the same in the input data group (38), and acquires an output data group (46) which are statistical data of the predicted result of the treatment action indicated by the input treatment information (44).

## Description

### TECHNICAL FIELD

The present specification discloses a statistical data acquisition apparatus, a degree of contribution calculating apparatus, a therapeutic action searching apparatus, a therapy target searching apparatus, a statistical data acquisition program, a degree of contribution calculation program, a therapeutic action searching program, and a therapy target searching program.

### BACKGROUND

Learners have been used in the medical field. Non Patent Document 1, for example, discloses a system that outputs objective scores representing drugs to be administered to a patient and the efficacy thereof, based on patient information regarding cancer, genes, and variation, for example. Non Patent Document 2 discloses that AI (Artificial Intelligence) suggests possible disease names and proposed therapy methods based on examination data and data on symptoms.

### CITATION LIST

### NON PATENT LITERATURE

[Non-Patent Document 1] "ZD Net Japan", Aichi Cancer Center and Fujitsu developing AI-aided system for selecting drugs for each patient, https://japan.zdnet.com/article/35178336/
[Non-Patent Document 2] "ERP NAVI", No. 105, "Al" in medical care, https://www.otsuka-shokai.co.jp/erpnavi/topics/column/medical/ai-in-healthcare.html

### SUMMARY

### TECHNICAL PROBLEM

Bringing a certain therapeutic action into practical use requires statistical data consisting of an enormous amount of data showing results of the therapeutic action. Acquisition of an enormous amount of data requires significant costs, labor, or time, and this has been a bottleneck in practical use of therapeutic actions. The therapeutic action as used in the present specification refers to an action performed based on medical care for therapy, diagnosis, or prevention of diseases and injuries of human beings or animals. Because the therapeutic action includes administration of pharmaceuticals to human beings or animals, results of the therapeutic action can indicate efficacy and safety of the pharmaceuticals. In particular, when the therapeutic action intended for practical use is a therapeutic action performed on human patients, the statistical data includes statistical data for clinical trials. When the therapeutic action intended for practical use is a therapeutic action with the use of pharmaceuticals or medical devices, the statistical data can include statistical data for clinical study.

Further, when attribute information representing attributes of a therapy target (such as physical features or health conditions) or therapy information representing the content of the therapeutic action includes a plurality of data items, it may be effective to understand how each data item affects the result of the therapeutic action.

It may be also effective to specify a therapeutic action that is suitable for a certain therapy target.

The present disclosure is therefore aimed toward reducing costs, labor, or time required for acquiring statistical data indicating results of therapeutic actions. Alternatively, the present disclosure is aimed toward enabling simple understanding of how each of data items of attribute information of a therapy target and each of data items of therapy information affect the result of a therapeutic action. Alternatively, the present disclosure is aimed toward proposing a therapeutic action suitable for a certain therapy target.

### SOLUTION TO PROBLEM

A statistical data acquisition apparatus disclosed in the present specification includes a statistical data acquisition unit. The statistical data acquisition unit is configured to input an input data set to a learner. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. The input data set includes a plurality of input data elements including input attribute information representing an attribute of a therapy target and input therapy information representing a content of a therapeutic action with respect to the therapy target. The input data elements of the input data set have mutually different input attribute information and the input data elements of the input data set have identical input therapy information. The statistical data acquisition unit thereby acquires statistical data of a prediction result of a therapeutic action indicated by the input therapy information.

The input attribute information may have a value in accordance with a predetermined distribution.

The statistical data acquisition apparatus may further include a distribution adjusting unit. The distribution adjusting unit is configured to adjust, based on a plurality of distributions for determining values of the input attribute information, the statistical data corresponding to output data of the learner that has been trained, wherein the output data has been obtained in response to the input data elements including the input attribute information in accordance with the respective distributions, and target statistical data that is a target of a user, the distributions such that the statistical data output from the trained learner approaches the target statistical data.

The statistical data acquisition unit may be configured to acquire an output error distribution that is a distribution of an output error of the trained learner, and, based on the output error distribution, correct a result of the therapeutic action indicated by the input therapy information.

The learner may be configured to be trained with virtual learning data generated based on statistical information regarding a therapeutic action performed in the past.

The learner may be configured to be trained with virtual learning data indicated by statistical information regarding a therapeutic action performed in the past. The virtual learning data has been generated to conform to a distribution regarding the therapeutic action.

The learner may be configured to be trained with a first learning data set, and thereafter re-trained with a second learning data set that is different from the first learning data set.

Further, a degree of contribution calculating apparatus disclosed in the present specification includes a degree of contribution calculating unit. The degree of contribution calculating unit is configured to input, to a learner, first input data and second input data. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. The first input data includes first input attribute information representing an attribute of a therapy target and first input therapy information representing a content of a therapeutic action with respect to the therapy target, and the degree of contribution calculating apparatus inputs the first input data to the learner to thereby acquire a first prediction result. The second input data includes second input attribute information and second input therapy information having a plurality of data items of the first input attribute information and the first input therapy information, with one of the plurality of data items having been changed, and the degree of contribution calculating apparatus inputs the second input data to the learner to thereby acquire a second prediction result. The degree of contribution calculating unit thereby calculates a degree of contribution of the data items regarding the output of the learner based on a difference between the first prediction result and the second prediction result.

Further, a therapeutic action searching apparatus disclosed in the present specification includes a therapeutic action searching unit. The therapeutic action searching unit is configured to search for a therapeutic action suitable for a predetermined therapy target, based on prediction results of a plurality of mutually different therapeutic actions with respect to the predetermined therapy target. The prediction results have been acquired by inputting a plurality of input data elements to a learner. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. Each of the input data elements includes input attribute information representing an attribute of a therapy target and input therapy information representing a content of a therapeutic action with respect to the therapy target. The input data elements include the input therapy information elements that are mutually different.

Further, a therapeutic target searching apparatus disclosed in the present specification includes a therapeutic target searching unit. The therapeutic target searching unit is configured to search for a therapeutic target suitable for a predetermined therapy action, based on prediction results of the predetermined therapeutic action with respect to a plurality of mutually different therapy targets. The prediction results have been acquired by inputting a plurality of input data elements to a learner. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. Each of the input data elements includes input attribute information representing an attribute of a therapy target and input therapy information representing a content of a therapeutic action with respect to the therapy target. The input data elements include the input therapy information elements that are mutually different.

Further, a statistical data acquisition program disclosed in the present specification causes a computer to function as a statistical data acquisition unit. The statistical data acquisition unit is configured to input an input data set to a learner. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. The input data set includes a plurality of input data elements including input attribute information representing an attribute of a therapy target and input therapy information representing a content of a therapeutic action with respect to the therapy target. The input data elements of the input data set have mutually different input attribute information and the input data elements of the input data set have identical input therapy information. The statistical data acquisition unit thereby acquires statistical data of a prediction result of a therapeutic action indicated by the input therapy information.

A degree of contribution calculating program disclosed in the present specification causes a computer to function as a degree of contribution calculating unit. The degree of contribution calculating unit is configured to input, to a learner, first input data and second input data. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. The first input data includes first input attribute information representing an attribute of a therapy target and first input therapy information representing a content of a therapeutic action with respect to the therapy target, and the degree of contribution calculating apparatus inputs the first input data to the learner to thereby acquire a first prediction result. The second input data includes second input attribute information and second input therapy information having a plurality of data items of the first input attribute information and the first input therapy information, with one of the plurality of data items having been changed, and the degree of contribution calculating apparatus inputs the second input data to the learner to thereby acquire a second prediction result. The degree of contribution calculating unit thereby calculates a degree of contribution of the data items regarding the output of the learner based on a difference between the first prediction result and the second prediction result.

Further, a therapeutic action searching program disclosed in the present specification causes a computer to function as a therapeutic action searching unit. The therapeutic action searching unit is configured to search for a therapeutic action suitable for a predetermined therapy target, based on prediction results of a plurality of mutually different therapeutic actions with respect to the predetermined therapy target. The prediction results have been acquired by inputting a plurality of input data elements to a learner. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. Each of the input data elements includes input attribute information representing an attribute of a therapy target and input therapy information representing a content of a therapeutic action with respect to the therapy target. The input data elements include the input therapy information elements that are mutually different.

A therapeutic target searching program disclosed in the present specification causes a computer to function as a therapeutic target searching unit. The therapeutic target searching unit is configured to search for a therapeutic target suitable for a predetermined therapy action, based on prediction results of the predetermined therapeutic action with respect to a plurality of mutually different therapy targets. The prediction results have been acquired by inputting a plurality of input data elements to a learner. The learner has been trained to predict and output, using learning data, from attribute information representing an attribute of a therapy target and therapy information representing a content of a therapeutic action with respect to the therapy target, a result of the therapeutic action with respect to the therapy target. The learning data includes learning attribute information representing an attribute of a past therapy target, learning therapy information representing a content of a therapeutic action with respect to the past therapy target, and a learning therapy result representing a result of the therapeutic action with respect to the past therapy target. Each of the input data elements includes input attribute information representing an attribute of a therapy target and input therapy information representing a content of a therapeutic action with respect to the therapy target. The input data elements include the input therapy information elements that are mutually different.

### ADVANTAGEOUS EFFECTS OF INVENTION

The present disclosure enables reductions in costs, labor, or time required for acquiring statistical data indicating results of therapeutic actions. The present disclosure further enables simple understanding of the effect of each data item of attribute information of a therapy target and each data item of therapy information, on the result of a therapeutic action. The present disclosure further enables proposal of a therapeutic action suitable for a certain therapy target.

### BRIEF DESCRIPTION OF DRAWINGS

[FIGURE 1] FIG. 1 is a diagram schematically illustrating a configuration of a server according to the present embodiment.
[FIGURE 2] FIG. 2 is a chart showing an example learning data set.
[FIGURE 3] FIG. 3 is a chart showing a first example input data set.
[FIGURE 4] FIG. 4 is a chart showing a second example input data set.
[FIGURE 5] FIG. 5 is a chart showing an example output data set.
[FIGURE 6] FIG. 6 shows distributions of each data item of input attribute information.
[FIGURE 7] FIG. 7 shows correction of each output data in accordance with an output error of a learner.
[FIGURE 8] FIG. 8 shows degrees of contribution of input attribute information and input therapy information to output data of each data item.
[FIGURE 9] FIG. 9 is a chart showing input data and output data of a learner for use in searching for therapy information.

### DESCRIPTION OF EMBODIMENTS

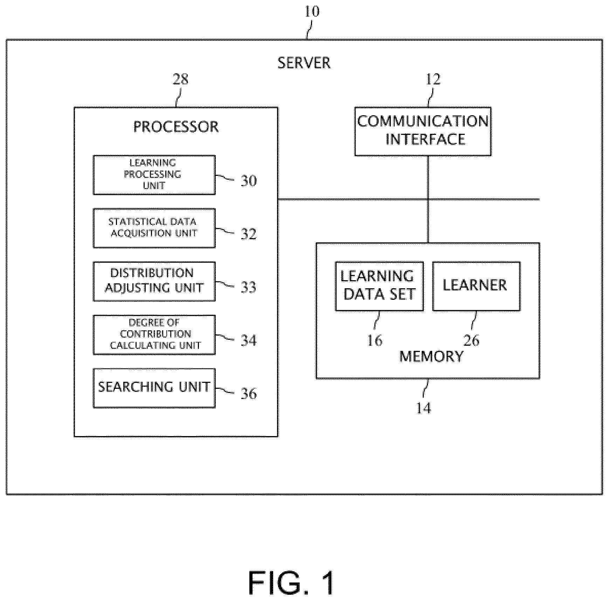

FIG. 1 is a diagram schematically illustrating a configuration of a server 10 that serves as a statistical data acquisition apparatus, a degree of contribution calculating apparatus, a therapeutic action searching apparatus, or a therapy target searching apparatus. The statistical data acquisition apparatus, the degree of contribution calculating apparatus, the therapeutic action searching apparatus, or the therapy target searching apparatus, which exerts functions described below, may be implemented by a computer other than the server 10. Each of the functions described below may also be implemented by collaboration of a plurality of computers.

A communication interface 12 is composed of an NIC (Network Interface Card) or a near field communication adaptor, for example. The communication interface 12 has a function to communicate with other computers. For example, the communication interface 12 is capable of receiving, from other computers, various data necessary for processing that will be described below. The communication interface 12 is further capable of transmitting, to other computers, information indicative of results of processing.

A memory 14 includes, for example, an HDD (Hard Disk Drive), SSD (Solid State Drive) eMMC (embedded Multi Media Card), ROM (Read Only Memory), or RAM (Random Access Memory). The memory 14 stores a computer program that serves as a statistical data acquisition program, a degree of contribution calculating program, a therapeutic action searching program, or a therapy target searching program, which causes each element of the server 10 to operate. These computer programs may also be stored in a computer-readable non-transitory storage medium, such as a USB (Universal Serial Bus) memory or a CD-ROM. The server 10 retrieves the computer program from such a storage medium, and executes the computer program.

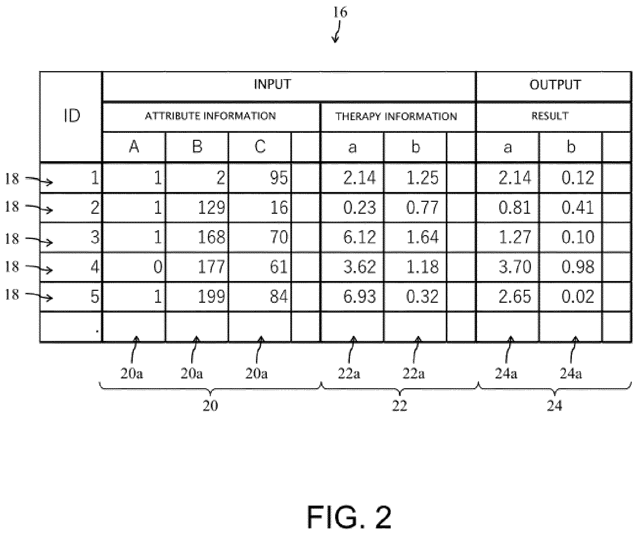

As illustrated in FIG. 1, the memory 14 stores a learning data set 16. The learning data set 16 is used to train a learner 26 that will be described below. FIG. 2 shows an example of the learning data set 16. The learning data set 16 consists of a plurality of learning data elements 18. Each learning data element 18 includes learning attribute information 20 representing attributes of a past therapy target, learning therapy information 22 representing the content of therapeutic actions for the therapy target, and a learning therapy result 24 representing results of the therapeutic actions. Here, the therapy targets include human beings or animals. The attributes of the therapy target include physical characteristics (such as sex, age, height, weight, blood type, underlying disease, and allergy), and health conditions (including psychological conditions) of the therapy target. The therapeutic action refers to an action performed based on medical care for therapy, diagnosis, or prevention of diseases and injuries of the therapy target, and includes administration of pharmaceuticals to human beings or animals. Among the learning data elements 18, the learning therapy result 24 corresponds to teaching data.

In the present embodiment, the learning attribute information 20 includes a plurality of data items 20a (e.g., parameters representing physical characteristics and health conditions). In the present embodiment, the learning therapy information 22 also includes a plurality of data items 22a (e.g., parameters representing therapy contents). In the present embodiment, the learning therapy result 24 also includes a plurality of data items 24a (e.g., parameters regarding therapy targets after therapy). The learning data elements 18 are to be input to the learner 26 or compared with the output data of the learner 26. Therefore, values of the data items 20a, 22a, and 24a are numerical values converted from data indicative of the attributes of therapy targets, the contents of the therapeutic actions, or results of the therapeutic actions.

The learning data set 16 is previously prepared by a user of the server 10, for example, and stored in the memory 14. Alternatively, the learning data set 16 may be stored in a memory of other apparatuses accessible from the server 10, in place of the memory 14.

Referring back to FIG. 1, the memory 14 further stores the learner 26. The learner 26 is a program for performing machine learning based on the learning data set 16. While the learner 26 is typically configured by a neural network, the learner 26 may have any other configuration that can perform the functions described below. The learner 26 may also be stored in a memory of an apparatus that is accessible from the server 10, in place of the memory 14. The learning method and processing content of the learner 26 will be described below in connection with a processor 28.

The processor 28 includes at least one of a general-purpose processor (e.g., CPU (Central Processing Unit)) and a dedicated processor (e.g., ASIC (Application Specific Integrated Circuit), FPGA (Field-Programmable Gate Array), or programmable logic apparatus). The processor 28 may be configured by collaboration of a plurality of processors that are physically separated from each other, rather than by a single processor. As illustrated in FIG. 1, the processor 28, in accordance with a computer program stored in the memory 14, functions as a learning processing unit 30, a statistical data acquisition unit 32, a degree of contribution calculating unit 34, and a searching unit 36.

The learning processing unit 30 executes learning processing to train the learner 26 with the learning data set 16. Specifically, the learning processing unit 30 inputs to the learner 26 the learning attribute information 20 and the learning therapy information 22, among the learning data elements 18 (see FIG. 2). The learner 26 predicts a result of a therapeutic action represented by the input learning therapy information 22, with respect to a therapy target represented by the input learning attribute information 20, and outputs the prediction result as output data. The learning processing unit 30 then calculates a difference between the output data and the learning therapy result 24 corresponding to the teaching data among the learning data elements 18, and adjusts the parameters of the learner 26 (e.g., the weight and bias of each neuron when the learner 26 is a neural network) to decrease the difference. The learning processing unit 30 repeats this processing to thereby train the learner 26.

The learning processing unit 30 may perform pre-processing to correct missing data, imbalance, or outliers, with respect to each learning data peace 18 included in the learning data set 16, and train the learner 26 using the pre-processed learning data set 16. When the data item 20a, the data item 22a, or the data item 24a of the learning data element 18 is missing, for example, the learning processing unit 30 may complement the missing data based on known document information. Pre-processing of the learning data elements 18 results in an increase in the learning efficiency of the learner 26.

The learning processing unit 30 may also correct the data item 20a, the data item 22a, or the data item 24a of the learning data element 18 with a correction model to estimate, from the result of a therapeutic action for a certain therapy target, a result of a therapeutic action for a therapy target other than the certain therapy target, based on the known document information. This enables acquisition of the learning data element 18 showing a result of clinical trials that are assumed to be performed in one country, based on the result of clinical trials that were performed in other countries, for example.

Further, documents, for example, may disclose statistical information regarding therapeutic actions performed in the past. The statistical information regarding therapeutic actions performed in the past includes statistical information regarding attributes of a therapy target related to the therapeutic actions, and statistical information regarding the therapy result of the therapeutic action. The statistical information regarding the attributes of the therapy target includes, for each attribute of the therapy targets (e.g., age, sex, and disease), a ratio of the therapy targets belonging to each class (e.g., when the attribute refers to an age, a class for 0 to 10 years old, a class for 11 to 20 years and so on), for example. Similarly, the statistical information regarding therapy result includes, for each attribute of the therapy results (e.g., efficacy, and variation of efficacies), a ratio of the therapy results belonging to each class. The learning processing unit 30 may generate the learning data elements 18 virtually, based on such statistical information regarding the therapeutic actions performed in the past, and then train the learner 26 with the virtual learning data elements 18.

In particular, it can be understood that the statistical information regarding therapeutic actions performed in the past indicates distributions regarding the therapeutic actions. The distribution regarding the therapeutic actions refers to a distribution of therapy targets of the therapeutic actions, and a distribution of therapy results of the therapeutic actions. The learning processing unit 30 may therefore generate virtual learning data 18 so as to correspond to the distribution regarding the therapeutic actions performed in the past. More specifically the learning processing unit 30 may generate the virtual learning data 18 in such a manner that the value of each piece of learning attribute information 20 (more specifically, each data item 20a) included in the virtual learning data 18 corresponds to the statistical information regarding the therapy target of the therapeutic action performed in the past and the value of each piece of learning therapy information 22 (more specifically, each data item 22a) included in the virtual learning data 18 corresponds to the statistical information regarding the therapy result of the therapeutic action performed in the past.

The learning processing unit 30 may further retrain the learner 26 with a first learning data set 16, and thereafter retrain the learner 26 with a second learning data set 16 that is different from the first learning data set 16. For example, the learning processing unit 30 may retrain the learner 26 with the first learning data set 16 representing the result of a therapeutic action performed in a first country, and thereafter further retrain the learner 26 having been trained with the first learning data set 16, with the second learning data set 16 representing the result of a therapeutic action performed in a second country. This can provide the learner 26 that has been sufficiently trained and finely-tuned for a specific country, with a small amount of learning data representing the result of a therapeutic action within the specific country.

The sufficiently trained learner 26 is capable of predicting, with high precision, from the attribute information representing the attribute of a therapy target and the therapy information representing the content of a therapeutic action for the therapy target, the result of the therapeutic action with respect to the therapy target, and outputting the result.

While in the present embodiment, the learning processing of the learner 26 is executed in the server 10, the learning processing of the learner 26 may be executed anywhere other than in the server 10. The sufficiently trained learner 26 may be stored in the memory 14 of other apparatuses. In this configuration, the processor 28 may omit the function of the learning processing unit 30.

The statistical data acquisition unit 32 inputs an input data set composed of a plurality of input data elements into the trained learner 26 (which may be hereinafter referred to simply as the "learner 26") to thereby acquire statistical data representing a prediction result of a certain therapeutic action.

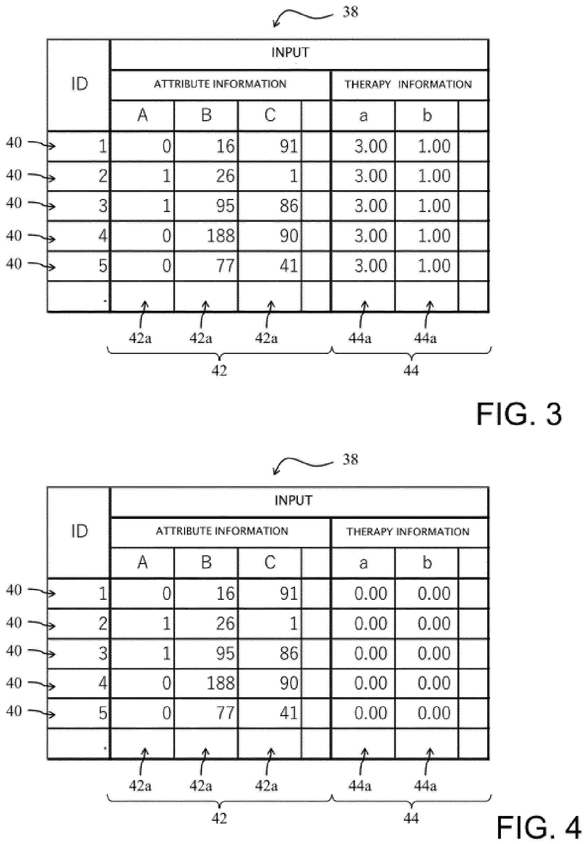

FIG. 3 and FIG. 4 show example input data sets 38 to be input to the learner 26. The input data set 38 includes a plurality of input data elements 40. Each input data element 40 includes input attribute information 42 representing attributes of a therapy target, and input therapy information 44 representing the contents of therapeutic actions for the therapy target. Similar to the learning data 18, in the present embodiment, the input attribute information 42 includes a plurality of data items 42a, and the input therapy information 44 also includes a plurality of data items 44a.

As shown in FIG. 3 and FIG. 4, in the input data set 38 to be input by the statistical data acquisition unit 32 to the learner 26, each input data element 40 includes different attribute information 42 and identical input therapy information 44.

The statistical data acquisition unit 32 is capable of random generation of the input attribute information 42 of each input data element 40. As described above, in the present embodiment, the input attribute information 42 includes a plurality of data items 42a, and the statistical data acquisition unit 32 is therefore capable of randomly generating each data item 42a.

Therapeutic actions desired by a user of the server 10 (a person who generates the statistical data) may be set as the therapeutic actions represented by the input therapy information 44. The therapeutic actions represented by the input therapy information 44 may include "performing no therapy". In this case, as shown in FIG. 4, the value of the input therapy information 44 (more specifically, each data item 44a) of each input data element 40 is set to "0.00", for example.

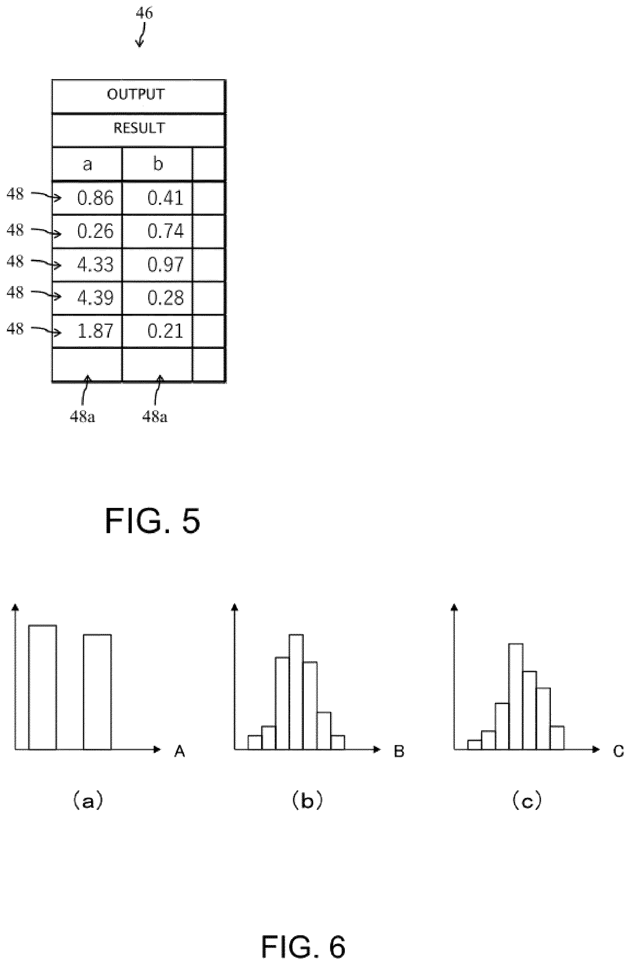

Receiving the input data set 38 as described above, the learner 26 outputs an output data set 46. FIG. 5 shows an example output data set 46 of the learner 26 in response to the input data set 38. The output data set 46 includes a plurality of output data elements 48. Each output data element 48 corresponds to data output by the learner 26 with respect to each input data element 40, and to data representing a prediction result of the therapeutic action for the therapy target indicated by each input data element 40. Similar to the learning data 18, each output data element 48 may include a plurality of data items 48a.

The output data set 46 corresponds to prediction results of the therapeutic actions acquired by performing the same therapeutic action with respect to a plurality of therapy targets having different attributes. In other words, the output data set 46 can be considered as statistical data of prediction results of the therapeutic actions represented by the input therapy information 44.

The present embodiment, in which the learner 26 is used to obtain the statistical data of the prediction results of the therapeutic actions, can reduce costs, labor, or time required for obtaining the statistical data, when compared to the prior art.
Conventionally, when a doctor, for example, wishes to acquire statistical data of results (efficacy and side effect, for example) of a new therapy method, the doctor actually repeats the new therapy method with respect to a large number of therapy targets to obtain the results, which requires enormous costs, labor, and time. According to the present embodiment, it is only necessary to prepare data representing results of therapeutic actions that enables sufficiently training for at least the learner 26 (that is, the learning data set 16). The input data set 38 is then input to the trained learner 26, which outputs the statistical data of the prediction results of the therapeutic action.

It is also possible to easily obtain the statistical data for either the case where a certain therapeutic action is performed or the case where the certain therapeutic action is not performed, and also to easily compare the prediction results between the case of performing a certain therapeutic action and the case of not performing the therapeutic action.

When the therapy target indicated by the input attribute information 42 is a human being, the statistical data can be statistical data for clinical trials. When the therapeutic action indicated by the input therapy information 44 is a therapeutic action with pharmaceuticals or with a medical device, the statistical data can be statistical data for clinical study. Conventionally, enormous costs, labor, or time are required to obtain statistical data that is needed for receiving approval of a pharmaceutical or a therapy method. In this regard, the statistical data according to the present embodiment, when accepted as the statistical data for approval, would enable simpler and earlier acquisition of the approval for new pharmaceutical and therapy method. In particular, in the case of sudden spread of a new infectious disease, for example, it is desired that a pharmaceutical or a therapy method against the infectious disease should be approved immediately. According to the present embodiment, it is possible to obtain the statistical data for approval of the pharmaceuticals immediately. Further, it is sometimes difficult to obtain a sufficient volume of data regarding the results (existence of efficacy and side effects) of pharmaceuticals and therapy methods for rare diseases, due to the small number of cases of these diseases. In such cases, the configuration of the present embodiment enables acquisition of the sufficient number of data for obtaining approval of the pharmaceuticals and the like for rare diseases.

The statistical data acquisition unit 32 may generate the value of the input attribute information 42 of each input data element 40 in accordance with a predetermined distribution. In the present embodiment, the input attribute information 42 includes a plurality of data items 42a. The statistical data acquisition unit 32 therefore generates the value of each data item 42a in accordance with the predetermined distribution.

FIG. 6 shows distributions of the data items 42a of the attribute information 42. FIG. 6(a) is a graph showing the distribution of the data items 42a labelled "A", FIG. 6(b) is a graph showing the distribution of the data items 42a labelled "B", and FIG. 6(c) is a graph showing the distribution of the data items 42a labelled "C". In each of the graphs of FIGs. 6(a) to 6(c), the horizontal axis indicates values of the data items 42a, and the vertical axis indicates the frequency of the values. These distributions are prestored in the memory 14 by the user of the server 10, for example. Alternatively, these distributions may be determined by a distribution adjusting unit 33, which will be described below.

The statistical data acquisition unit 32 randomly sets the value of each data item 42a of the input attribute information 42 so as to conform to the distributions shown in FIGs. 6(a) to 6(c), thereby generating the input data set 38. This enables the user of the server 10 to obtain the statistical data representing the prediction results of the therapeutic actions for a population of desired therapy targets.

Even the sufficiently trained learner 26 may also cause an output error. The output error refers to a difference between the output data 48 of the learner 26 in response to the certain input data 40, and a true (accurate) result of the therapeutic action for the therapy target indicated by the input data 40. The statistical data acquisition unit 32 may therefore acquire an output error distribution that is a distribution of the output errors of the learner 26, and correct the output data 48 based on the output error distribution.

The statistical data acquisition unit 32 first acquires a learning error distribution of the trained learner 26. The learning error distribution refers to information indicating a relation between the magnitude of an error of the learner 26 and the probability of occurrence of the error. The learning error distribution can be expressed by a graph in which the horizontal axis indicates the magnitude of an error and the vertical axis indicates the probability of occurrence of the error, for example.

The statistical data acquisition unit 32 acquires the learning error distribution by using a plurality of validation data sets (combinations of the attribute information representing attributes of therapy targets, the therapy information representing the content of the therapeutic actions for the therapy targets, and the therapy results representing the results of the therapeutic actions) that are different from the learning data set 18. Specifically, the statistical data acquisition unit 32 inputs the attribute information and the therapy information, among the validation data set, to the trained learner 26, and acquires a difference between the output data of the learner 26 in response to the input information and the therapy result of the validation data, as an error. The statistical data acquisition unit 32 performs this processing with regard to the plurality of validation data sets, thereby acquiring the learning error distribution of the learner 26. It is further possible to prepare a plurality of trained learners 26 (by training a plurality of learners 26 with the learning processing unit 30, for example), and calculate a statistic (e.g., a mean value or a standard deviation) of differences between the output data and the therapy results of the validation data with regard to the respective learners 26, to thereby acquire the learning error distribution.

The statistical data acquisition unit 32 corrects the output data 48 based on the acquired learning error distribution of the learner 26. For example, the statistical data acquisition unit 32 generates random numbers based on the learning error distribution, and adds the random numbers to the output data 48 to thereby correct the output data 48.

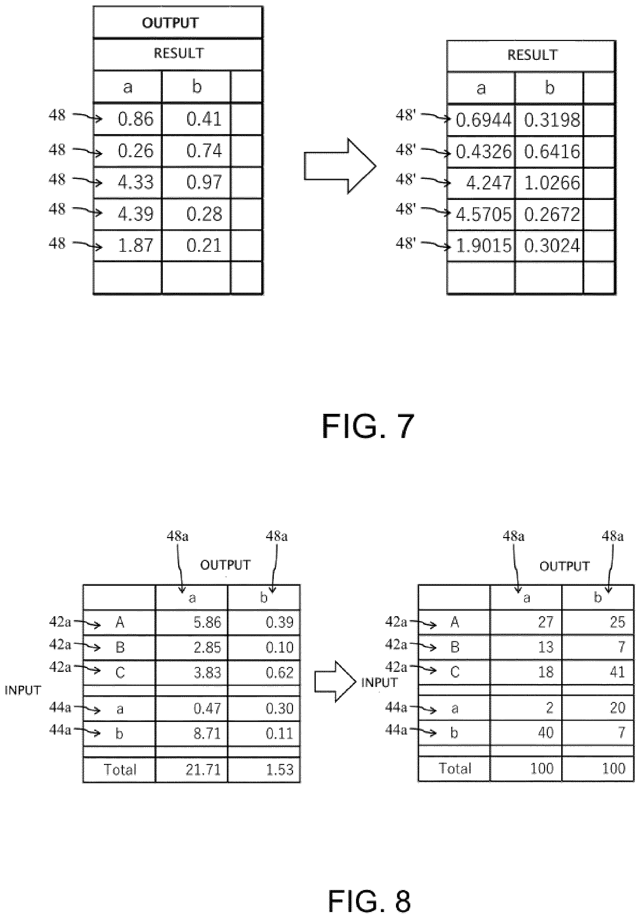

FIG. 7 shows the output data 48 before the correction and the corrected output data 48' obtained by correction based on the learning error distribution of the learner 26.

It is also possible to evaluate robustness of the learner 26 based on the learning error distribution, the output data 48, and the corrected output data 48' of the learner 26. The robustness of the learner 26 refers to an index indicating the degree of tolerance of the output data 48 (the prediction results of the therapeutic actions) against the output error of the learner 26.

Referring back to FIG. 1, the distribution adjusting unit 33 adjusts the distribution described above (see FIG. 6) that is referenced by the statistical data acquisition unit 32 to generate the values of the input attribute information 42 (more specifically, each data item 42a) of each input data set 40 input to the trained learner 26.

As a precondition, the output data of the trained learner 26; that is, the statistical data of the prediction result of the therapeutic actions, may vary in accordance with the distribution for determining the values of the input attribute information 42. Further, the statistical data of the prediction result of the therapeutic actions include ideal statistical data (e.g., when the therapeutic action is administration of drug, statistical data with a large mean value of the efficacies of drug administered to the therapy targets and with a small variation in the efficacy). The ideal data can be regarded as target statistical data intended by the user of the server 10.

Therefore, the distribution adjusting unit 33 may, based on a plurality of distributions for determining the values of the attribute information 42, the statistical data or the output data of the trained learner 26 in response to the input data including the input attribute information 42 in accordance with the respective distributions, and the target statistical data intended by the user, adjust the distributions, such that the statistical data output by the trained learner 26 approaches the intended statistical data. Here, any processing that adjusts the distributions for determining the values of the input attribute information 42, which searches for distributions that enable the trained learner 26 to output the intended statistical data, may be referred to as optimization of distributions. However, the optimization processing is not necessarily restricted to processing for acquiring an optimal solution (in this example, a distribution that can provide the intended statistical data), and refers to processing for searching toward the optimal solution.

Various methods may be employed for the optimization. For example, the distribution adjusting unit 33 uses a genetic algorithm to optimize the distribution for determining the values of the input attribute information 42. The genetic algorithm refers to the following processing. First, a plurality of "individuals" (also referred to as "chromosomes") that are optimization targets are randomly prepared. An individual is composed of a plurality of parameters called "genes". These plural individuals are referred to as individuals of a first generation. A predetermined evaluation function is employed to calculate a parameter called "fitness" for each of the individuals of the first generation. Then, based on the calculated fitness of each individual, genetic operation is performed for the plurality of individuals of the first generation. The genetic operation includes, for example, "selection" for selecting an individual according to the fitness, "crossover" for exchanging genes among a plurality of individuals, and "mutation" for changing part of a gene of an individual. The plurality of individuals that have undergone the genetic operation belong to the next generation (in this example, the second generation). Then, the fitness is calculated for each individual of the second generation. This processing is repeated until a predetermined termination condition (that a specific number of generations is reached, or that the mean value of the levels of fitness of a plurality of individuals of a certain generation reaches a predetermined value or more). This results in acquisition of individuals with high fitness.

More specifically, the distribution adjusting unit 33 inputs, to the trained learner 26, each of the input data sets including a plurality of pieces of input attribute information 42 (which correspond to the "individuals" of the first generation) generated based on a plurality of initial distributions. The learner 26 outputs the statistical data of the prediction result of the therapeutic actions in response to the respective input data sets corresponding to the respective distributions. The distribution adjusting unit 33 then performs statistical analysis with respect to the plurality of statistical data sets (a plurality of statistical data sets corresponding to the respective distributions) thus obtained. When the therapeutic action is administration of drug, for example, the distribution adjusting unit 33 acquires the mean value of efficacies or a variation of efficacies for each statistical data set. The result of the statistical analysis (i.e., the mean value of efficacies, or a variation of efficacies) corresponds to the "fitness" in the genetic algorithm. In this example, it is assumed that the intended statistical data corresponds to statistical data with the maximum mean value of efficacies and the minimum variation of efficacies. As such, in this example, the optimization processing for searching corresponds to processing for maximizing the mean value of efficacies and minimizing a variation of efficacies in the statistical data.

Thereafter, the distribution adjusting unit 33, based on the result of statistical analysis of the statistical data corresponding to each distribution, performs genetic operation with respect to a plurality of distributions of the first generation, to acquire a plurality of distributions of the second generation. Such processing is repeated to thereby optimize the distributions for determining the values of the input attribute information 42.

In actual clinical study, in terms of cost reduction, there is a demand for reducing the clinical study data (combinations of information indicating a target of clinical study and information indicating the therapy efficacy of the corresponding therapeutic action) as much as possible. Here, targets of clinical study in accordance with the optimized distribution, which enables acquisition of the intended statistical data described above, are also advantageous in that the number of targets (i.e., the number of clinical study data) can be reduced. This is because the more ideal the statistical data of the result of the therapeutic action, the greater the extent to which the volume of clinical study data can be reduced. For example, the greater the mean value of efficacies, or the smaller the variation of efficacy, the smaller the volume of clinical study data.

The degree of contribution calculating unit 34 calculates, based on differences among the plurality of output data 48 output from the trained learner 26 for the plurality of input data 40, degrees of contribution, regarding the data items 42a of the input attribute information 42 or the data items 44a of the input therapy information 44, included in the input data 40. The degrees of contribution indicate the degrees of contributions of the data items 42a and 44a to the output data 48.

The degree of contribution calculating unit 34 first selects a first input data element 40 from the input data set 38 (see FIG. 3). The input attribute information 42 of the first input data element 40 will be referred to as first input attribute information 42, and the input therapy information 44 of the first input data element 40 will be referred to as first input therapy information 44. The degree of contribution calculating unit 34 inputs the first input data element 40 to the learner 26 and acquires a first output data element 48 as a first prediction result. The degree of contribution calculating unit 34 then minutely changes one of the plurality of data items 42a and 44a included in the first input attribute information 42 and the first input therapy information 44, to obtain a second input data element 40 including second input attribute information 42 and second input therapy information 44. The degree of contribution calculating unit 34 further inputs the second input data element 40 to the learner 26, and acquires a second output data element 48 as a second prediction result. The degree of contribution calculating unit 34 then calculates a difference between the first output data element 48 and the second output data element 48.

The degree of contribution calculating unit 34 calculates the difference between the first output data element 48 and the second output data element 48 while varying the data item 42a, 44a to be changed, to obtain such differences with each of the data items 42a and 44a being changed (hereinafter referred to simply as a "difference regarding each data item 42a, 44a").

The chart to the left of an arrow in FIG. 8 shows absolute values of differences regarding each data item 42a, 44a. In the present embodiment, the degree of contribution calculating unit 34 calculates, regarding each data item 42a, 44a, an absolute value of the difference for each data item 48a of the output data 48, and FIG. 8 shows the result. Further, as illustrated in FIG. 8, the degree of contribution calculating unit 34 calculates, for each data item 48a of the output data 48, a sum of the absolute values of differences of each data item 42a, 44a. In the example shown in FIG. 8, the sum is "21.71" for the data item 48a labeled "a", and the sum is "1.53" for the data item 48a labeled "b".

The chart to the right of the arrow in FIG. 8 shows the degree of contribution of each data item 42a, 44a to each output data element 48 obtained based on the chart on the left. The degree of contribution calculating unit 34 normalizes the absolute values of the differences regarding each data item 42a, 44a such that the sum of the absolute values of the respective data items 48a of the output data 48 is "100". The normalized values are shown in the right chart in FIG. 8, in which the normalized numeral value represents the degree of contribution of each data item 42a, 44a to the output data 48. In the present embodiment, the degree of contribution of each data item 42a, 44a is shown for each data item 48a of the output data 48.

While in the present embodiment, the degree of contribution is calculated based on the absolute value of the difference regarding the data items 42a and 44a, the degree of contribution of each data item 42a, 44a may be calculated separately for the differences between the first output data 48 and the second output data 48 having a positive value and a negative value. The degree of contribution calculate unit 34 may further perform statistical analysis (e.g., t-test or p-test) regarding the degree of contribution of each data item 42a, 44a to thereby verify the statistical likelihood thereof.

Referring back to FIG. 1, the searching unit 36 inputs the plurality of input data elements 40 including different input therapy information 44 to the trained learner 26, and, based on a plurality of output data elements 48 of the learner 26 obtained in response; that is, the prediction results of a plurality of therapeutic actions for a predetermined therapy target, searches for a therapeutic action suitable for the predetermined therapy target. As such, the searching unit 36 functions as a therapeutic action searching unit.

Regarding the processing for searching for the therapeutic action, the processing for searching for an optimal therapeutic action for a predetermined therapy target may be referred to as the optimization processing of the therapeutic action. However, the optimization processing is not necessarily limited to the processing for obtaining an optimal solution (in this example, an optimal therapeutic action), and refers to processing for searching toward an optimal solution. The optimal solution may be a certain fixed value or may indicate a range with a certain degree of width.

While various methods may be employed for optimization processing of the therapeutic action using the learner 26, optimization processing using a genetic algorithm will be described below.

In a genetic algorithm that searches for the therapeutic action, an "individual" corresponds to the input therapy information 44, a "gene" corresponds to the data item 44a of the input therapy information 44, and a "fitness" is calculated based on the output data 48 of the learner 26 in response to the input data 40 including the input therapy information 44 as an individual.

The genetic algorithm that searches for the therapeutic action will be specifically described. First, a plurality of input data elements 40 including the same input attribute information 42 and different input therapy information 44 are prepared. Here, the therapy target indicated by the input attribute information 42 may be a therapy target desired by the user of the server 10. For example, an attribute of a patient to which a therapy method to be searched for is applied is set. The searching unit 36 may randomly generate the input therapy information 44 of each input data element 40. The plurality of input therapy information 44 included in the plurality of input data 40 thus prepared correspond to the individuals of the genetic algorithm, and each data item 44a included in the input therapy information 44 corresponds to the gene of the genetic algorithm.

The searching unit 36 then sequentially inputs the plurality of input data elements 40 to the learner 26, and acquires the plurality of output data elements 48 corresponding to the respective input data elements 40. The searching unit 36 calculates a difference between ideal result data obtained by digitizing the ideal therapeutic action result into the form of an output data element 48, and each output data 48. The searching unit 36 then calculates, based on the difference, a difference parameter that is a parameter that increases as the difference is smaller and decreases as the difference is greater. The difference parameter is a parameter with respect to the input data 40 (particularly the input therapy information 44), and corresponds to the fitness of the genetic algorithm.

The searching unit 36, based on the difference parameter that is a fitness of the genetic algorithm, regarding each piece of input therapy information 44 that is an individual of the genetic algorithm, executes genetic operation for each piece of input therapy information 44. For example, the searching unit 36 performs operation such as selection, crossover, or mutation for each data item 44a included in each piece of input therapy information 44. This operation results in generation of a plurality of input data elements 40 including the input therapy information 44 of the next generation (the second generation in this example). Here, the searching unit 36 does not change the input attribute information 42. In other words, the input attribute information 42 is fixed.

Thereafter, the searching unit 36 repeats the above processing. Specifically, the searching unit 36 inputs the plurality of input data elements 40 including the input therapy information 44 of the second generation to the learner 26, and, based on the difference parameter of each input therapy information 44 obtained from the output data 48 in response to the input data 40, performs genetic operation for each piece of input therapy information 44. The searching unit 36 thereby generates a plurality of input data elements 40 including input therapy information 44 of a further next generation.

The searching unit 36 repeats the above processing until a predetermined termination condition is satisfied. The searching unit 36 then specifies, as a therapeutic action suitable for the therapy target, a therapeutic action represented by the input therapy information 44 having the greatest difference parameter, among the plurality of pieces of input therapy information 44 obtained at the time of termination.

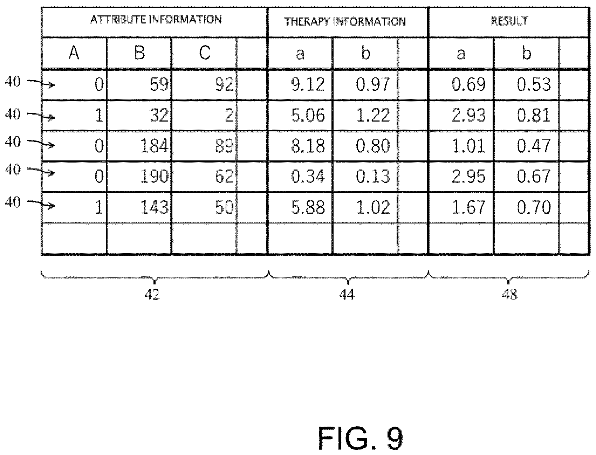

The plurality of input data elements 40 including a single type of input attribute information 42 or the plurality of input data elements 40 including a plurality of mutually different input attribute information 42 may be used in the genetic algorithm described above. The plurality of attribute information 42 may be determined in accordance with the predetermined distribution (see FIG. 6) or a distribution adjusted by the distribution adjusting unit 33. Examples of the plurality of input data elements 40 in this case are shown in FIG. 9. The basic processing is also the same as that described above, and the plurality of pieces of input attribute information 42 remain unchanged during the genetic algorithm. However, the difference parameter that is the fitness is calculated based on a plurality of differences between the ideal result data and each output data 48 in response to each piece of input data 40. For example, the difference parameter is calculated based on the mean value of the plurality of differences.

The searching unit 36 may further input the plurality of input data elements 40 including different input attribute information 42 to the trained learner 26, and, based on the plurality of output data elements 48 of the learner 26 obtained in response; that is, the prediction results of a predetermined therapeutic action for a plurality of different therapy targets, search for a therapy target suitable for the predetermined therapeutic action. As such, the searching unit 36 also functions as a therapy target searching unit.

The processing for searching for a therapy target, which searches for an optimal therapy target for a predetermined therapeutic action, can also be referred to as optimization of a therapy target.

While various methods for optimization of a therapy target using the learner 26 may be employed, again, the optimization processing with a genetic algorithm will be described.

In a genetic algorithm that searches for a therapy target, an "individual" corresponds to the input attribute information 42, a "gene" corresponds to the data item 42a of the input attribute information 42, and a "fitness" is calculated based on the output data 48 of the learner 26 in response to the input data 40 including the input attribute information 42 as an individual.

The genetic algorithm that searches for a therapy target will be specifically described. First, a plurality of input data elements 40 including different input attribute information 42 and identical input therapy information 44 are prepared. Here, the therapeutic action indicated by the input attribute information 42 may be a therapeutic action desired by the user of the server 10. The searching unit 36 may randomly generate the input attribute information 42 of each input data element 40. The plurality of pieces of input attribute information 42 included in the plurality of input data elements 40 thus prepared correspond to the individuals of the genetic algorithm, and each data item 42a included in the input attribute information 42 corresponds to the gene of the genetic algorithm.

The searching unit 36 then sequentially inputs the plurality of input data elements 40 to the learner 26, and acquires a plurality of output data elements 48 corresponding to the respective input data elements 40. The searching unit 36 calculates a difference between ideal result data obtained by digitizing the ideal therapeutic action result into the form of an output data element 48, and each output data element 48. The searching unit 36 then calculates, based on the difference, a difference parameter that is a parameter that increases as the difference is smaller and decreases as the difference is greater. The difference parameter is a parameter with respect to the input data elements 40 (particularly the input attribute information 42), and corresponds to the fitness of the genetic algorithm.

The searching unit 36, based on the difference parameter that represents a fitness of the genetic algorithm, regarding each input attribute information 42 that is an individual of the genetic algorithm, executes genetic operation for each input attribute information 42. For example, the searching unit 36 performs operation such as selection, crossover, or mutation for each data item 42a included in each input attribute information 42. This operation results in generation of a plurality of input data elements 40 including the input attribute information 42 of the next generation (the second generation in this example). Here, the searching unit 36 does not change the input therapy information 44. In other words, the input therapy information 44 is fixed.

Thereafter, the searching unit 36 repeats the above processing. Specifically, the searching unit 36 inputs the plurality of input data elements 40 including the input attribute information 42 of the second generation to the learner 26, and, based on the difference parameter of each input attribute information 42 obtained from the output data elements 48 in response to the input data elements 40, performs genetic operation for each piece of input attribute information 42. The searching unit 36 thereby generates a plurality of input data elements 40 including input attribute information 42 of a further next generation.

The searching unit 36 repeats the above processing until a predetermined termination condition is satisfied. The searching unit 36 then specifies, as a therapy target suitable for the therapeutic action, a therapy target represented by input attribute information 42 indicating the greatest difference parameter, among the plurality of pieces of input attribute information 42 obtained at the time of termination.

Here, the plurality of input data elements 40 including a single type of input therapy information 44 or the plurality of input data elements 40 including a plurality of mutually different types of input therapy information 44 may be used in the genetic algorithm described above. The plurality of pieces of input therapy information 44 may be determined in accordance with the predetermined distribution (see FIG. 6) or a distribution adjusted by the distribution adjusting unit 33. In this case, the basic processing is also the same as that described above, and the plurality of pieces of input therapy information 44 remain unchanged during the genetic algorithm. However, the difference parameter that is the fitness is calculated based on a plurality of differences between the ideal result data and each output data element 48 in response to each input data element 40. For example, the difference parameter is calculated based on the mean value of the plurality of differences.

As described above, the searching unit 36 searches for a therapeutic action suitable for a predetermined therapy target, or a therapy target suitable for a predetermined therapeutic action. The searching unit 36 may search for a therapeutic action suitable for a predetermined therapy target, and a therapy target suitable for a predetermined therapeutic action.

While an embodiment according to the disclosure has been described above, the present disclosure is not limited to the above embodiment and various modifications may be made without departing from the gist of the disclosure.

### REFERENCE SIGNS LIST

10 server, 12 communication interface, 14 memory, 16 learning data set, 18 learning data, 20 learning attribute information, 20a, 22a, 24a, 42a, 44a, 48a data item, 22 learning therapy information, 24 learning therapy result, 26 learner, 28 processor, 30 learning processing unit, 32 statistical data acquisition unit, 33 distribution adjusting unit, 34 degree of contribution calculating unit, 36 searching unit, 38 input data set, 40 input data, 42 input attribute information, 44 input therapy information, 46 output data set, 48 output data, 48' corrected output data.

## Claims

1. A statistical data acquisition apparatus comprising:
a statistical data acquisition unit configured to input an input data set to a learner, the learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of a therapy target and therapy information representing a content of a therapeutic action with respect to the therapy target, a result of the therapeutic action with respect to the therapy target, wherein the input data set includes a plurality of input data elements including input attribute information representing an attribute of a therapy target and input therapy information representing a content of a therapeutic action with respect to the therapy target, the input data elements of the input data set having mutually different input attribute information and the input data elements of the input data set having identical input therapy information, the statistical data acquisition unit thereby acquiring statistical data of a prediction result of a therapeutic action indicated by the input therapy information.

2. The statistical data acquisition apparatus according to claim 1, wherein
the input attribute information has a value in accordance with a predetermined distribution.

3. The statistical data acquisition apparatus according to claim 1, further comprising:
a distribution adjusting unit configured to adjust, based on a plurality of distributions for determining values of the input attribute information, the statistical data corresponding to output data of the learner that has been trained, the output data having been obtained in response to the input data elements including the input attribute information in accordance with the respective distributions, and target statistical data that is a target of a user, the predetermined distribution such that the statistical data output from the trained learner approaches the target statistical data.

4. The statistical data acquisition apparatus according to claim 1, wherein
the statistical data acquisition unit is configured to acquire an output error distribution that is a distribution of an output error of the trained learner, and, based on the output error distribution, correct a result of the therapeutic action indicated by the input therapy information.

5. The statistical data acquisition apparatus according to claim 1, wherein
the learner is configured to be trained with virtual learning data generated based on statistical information regarding a therapeutic action performed in the past.

6. The statistical data acquisition apparatus according to claim 5, wherein
the learner is configured to be trained with virtual learning data indicated by statistical information regarding a therapeutic action performed in the past, the virtual learning data having been generated to conform to a distribution regarding the therapeutic action.

7. The statistical data acquisition apparatus according to claim 1, wherein
the learner is configured to be trained with a first learning data set, and is thereafter re-trained with a second learning data set that is different from the first learning data set.

8. A degree of contribution calculating apparatus, comprising:
a degree of contribution calculating unit configured to input, to a learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of a therapy target and therapy information representing a content of a therapeutic action with respect to the therapy target, a result of the therapeutic action with respect to the therapy target, first input data including first input attribute information representing an attribute of a therapy target and first input therapy information representing a content of a therapeutic action with respect to the therapy target to thereby acquire a first prediction result, and to input, to the learner, second input data including second input attribute information and second input therapy information having a plurality of data items of the first input attribute information and the first input therapy information, one of the plurality of data items having been changed, to thereby acquire a second prediction result, the degree of contribution calculating unit thereby calculating a degree of contribution of the data items regarding the output of the learner based on a difference between the first prediction result and the second prediction result.

9. A therapeutic action searching apparatus comprising:
a therapeutic action searching unit configured to search for a therapeutic action suitable for a predetermined therapy target, based on prediction results of a plurality of mutually different therapeutic actions with respect to the predetermined therapy target, the prediction results having been acquired by inputting a plurality of input data elements to a learner, the learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of a therapy target and therapy information representing a content of a therapeutic action with respect to the therapy target, a result of the therapeutic action with respect to the therapy target, wherein each of the input data elements includes input attribute information representing an attribute of a therapy target and input therapy information representing a content of a therapeutic action with respect to the therapy target, the input data elements including the input therapy information elements that are mutually different.

10. A therapy target searching apparatus comprising:
a therapy target searching unit configured to search for a therapy target suitable for a predetermined therapeutic action, based on prediction results of the predetermined therapeutic action with respect to a plurality of mutually different therapy targets, the prediction results having been acquired by inputting a plurality of input data elements to a learner, the learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of the therapy target and therapy information representing a content of a therapeutic action with respect to the therapy target, a result of the therapeutic action with respect to the therapy target, wherein each of the input data elements includes input attribute information representing an attribute of a therapy target and input therapy information representing a content of a therapeutic action with respect to the therapy target, the input data elements including the input attribute information elements that are mutually different.

11. A statistical data acquisition program causing a computer to function as a statistical data acquisition unit configured to input an input data set to a learner, the learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of a therapy target and therapy information representing a content of a therapeutic action with respect to the therapy target, a result of the therapeutic action with respect to the therapy target, wherein the input data set includes a plurality of input data elements including input attribute information representing an attribute of a therapy target and input therapy information representing a content of a therapeutic action with respect to the therapy target, each of the input data elements of the input data set having different input attribute information and each of the input data elements of the input data set having identical input therapy information, the statistical data acquisition unit thereby acquiring statistical data of a prediction result of a therapeutic action indicated by the input therapy information.

12. A degree of contribution calculation program causing a computer to function as a degree of contribution calculating unit configured to input, to a learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of a therapy target and therapy information representing a content of a therapeutic action with respect to the therapy target, a result of the therapeutic action with respect to the therapy target, first input data including first input attribute information representing an attribute of a therapy target and first input therapy information representing a content of a therapeutic action with respect to the therapy target to thereby acquire a first prediction result, and to input, to the learner, second input data including second input attribute information and second input therapy information having a plurality of data items of the first input attribute information and the first input therapy information, one of the plurality of data items having been changed, to thereby acquire a second prediction result, the degree of contribution calculating unit thereby calculating a degree of contribution of the data items regarding the output of the learner based on a difference between the first prediction result and the second prediction result.

13. A therapeutic action searching program causing a computer to function as a therapeutic action searching unit configured to search for a therapeutic action suitable for a predetermined therapy target, based on prediction results of a plurality of mutually different therapeutic actions with respect to the predetermined therapy target, the prediction results having been acquired by inputting a plurality of input data elements to a learner, the learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of a therapy target and therapy information representing a content of a therapeutic action with respect to the therapy target, a result of the therapeutic action with respect to the therapy target, wherein each of the input data elements includes input attribute information representing an attribute of a therapy target and input therapy information representing a content of a therapeutic action with respect to the therapy target, each of the input data elements including the input therapy information elements that are mutually different.

14. A therapy target searching program causing a computer to function as a therapy target searching unit configured to search for a therapy target suitable for a predetermined therapeutic action, based on prediction results of the predetermined therapeutic action with respect to a plurality of mutually different therapy targets, the prediction results having been acquired by inputting a plurality of input data elements to a learner, the learner having been trained to predict and output, using learning data including learning attribute information representing an attribute of a past therapy target, learning therapy information representing a content of a therapeutic action with respect to the past therapy target, and a learning therapy result representing a result of the therapeutic action with respect to the past therapy target, from attribute information representing an attribute of the therapy target and therapy information representing a content of a therapeutic action with respect to the therapy target, a result of the therapeutic action with respect to the therapy target, wherein each of the input data elements includes input attribute information representing an attribute of a therapy target and input therapy information representing a content of a therapeutic action with respect to the therapy target, each of the input data elements including the input attribute information elements that are mutually different.
